# EUROPEAN PATENT SPECIFICATION

(11) **EP 3 503 720 B1**
(45) Date of publication and mention of the grant of the patent: **25.11.2020**
(21) Application number: 17755288.2
(22) Date of filing: 22.08.2017
(51) Int. Cl.: A01K 29/00, A61B 5/103, A61B 5/107, A61B 5/11

(54) **METHOD AND DEVICE TO DETECT LAMENESS OF A COW**
VERFAHREN UND GERÄT ZUM DETEKTIEREN VON LAHMHEIT EINER KUH
MÉTHODE ET APPAREIL DE DÉTECTION DE BOITERIE D'UNE VACHE

(30) Priority: 25.08.2016 NL 2017365
(43) Date of publication of application: 03.07.2019
(73) Proprietor: Lely Patent N.V., 3147 PB Maassluis (NL)
(72) Inventor: SONG, Xiangyu, 3147 PB Maassluis (NL)
(74) Representative: Octrooibureau Van der Lely N.V.
(86) International application number: PCT/NL2017/050548
(87) International publication number: WO 2018/038604

(56) References cited:
- DE-B3-102015 004 451
- US-A1- 2005 257 748
- US-A1- 2016 073 614

## Description

The invention relates to a method to automatically detect lameness of a cow and a device to detect lameness of a cow.

WO 2013/052001 discloses a device and method for detecting lameness of a standing cow. The device comprises at least one optical imaging device and a processing arrangement coupled with the optical imaging device. The optical imaging device is arranged in a position to capture at least one image showing the lower portions of at least one leg of the cow and to forward the image to the processing arrangement. The processing arrangement is, in turn, configured to analyze the image to determine a condition of lameness when said at least one leg is held in a raised position on or above ground level.

In an embodiment of this device, the system is proposed to be integrated in a milking or feeding stall with an automatic or semi-automatic milking system in order to detect lameness of a dairy animal.

The device and method of WO 2013/052001 is based on the observation that when an animal is or is becoming lame, it tends to slightly lift the leg in question so as not to put weight on it when standing. By detecting whether a leg is held in a position, that differs from the normal flat standing position, whether in contact with a floor surface or held above this surface, the device is able to provide an early indicator of a possible lameness problem to the farmer or stockman enabling further investigation and treatment.

A drawback of the device and method of WO 2013/052001 is that the leg of the animal may move out of the field of view of the camera. Further, some legs may not be viewed with the camera. In order to view all legs at all times multiple cameras have to be provided.

Another drawback of the device and method of WO 2013/052001 is that the cameras have to be arranged at substantially the same height as the leg(s) of the cow. This height is also used to position other equipment, for example a milking robot or feeding device.

Also, at this height there is a considerable risk that dirt, such as manure, will fall onto the camera which may prevent proper functioning of the camera.

Also, document DE102015004451B3 discloses the preamble of the independent claims.

It is an object of the present invention to provide a method and device to detect lameness of a cow, lacking one or more of the above-mentioned drawbacks, or at least to provide an alternative method and/or device.
- The invention provides a method according to claim 1.

According to the method of the invention, the position of one or more reference locations of the barrel of the cow are used to determine at least one lameness parameter that is indicative for the lameness of the cow. The position of the one or more reference locations of the barrel may be determined on the basis of position related data obtained from the cow using a data acquisition system, for example a camera system. The position of the reference locations may be determined within any suitable coordinate system, for example with respect to a position of the data acquisition system, or another fixed location.

The position may for example be determined by calculating an x-coordinate, y-coordinate of the one or more reference locations of the barrel. The position of the may also include a z-coordinate of the one or more reference locations.

The method of the invention is based on the insight a cow suffering from lameness will have a way of standing or moving in a detection area substantially different from hoof-healthy animals. In particular, cows suffering from lameness will not stand still, but will show more movement in all directions of the detection area or the cow will lean against a side wall of the detection area. These movements or positions may be detected by determining one or more reference locations of the barrel of the cow. The barrel of the cow, also referred to as trunk or torso, is the main body part of the cow, excluding head, tail and legs.

An additional advantage of the method of the invention is that a cow suffering from lameness may be detected on the basis of a movement of the cow which may or may not entail lifting of the feet. Sometimes, cows suffering from lameness may be detected by determining shifting of weight to one side of the body without moving or lifting the problem leg, i.e. a postural sway. A drawback of the method of WO 2013/052001 is that in such case the lameness of the cow may not be detected. However, the method of the invention enables to detect movements of the barrel of the cow, even when the shifting movement of the cow does not include lifting of the respective foot or feet.

The acquisition of the position related data is carried out during the time period that the cow is present in the detection area. This recording may be during the complete time period, but also during a part thereof.

The detection area may be a confinement area in which the cow is confined during the time period. In this confinement area, the movement of the cow may be restricted within limits. However, the cow should have still some freedom for movement. For example, the cow should still be able to make a step forward or back or make a postural sway, i.e. a movement of the barrel of the cow without moving the feet. The confinement area is for example a milking box of a milking system or a feeding box. The confinement may also be the result of a body part of the cow being held at a fixed location, for example the neck of a cow may be held by a fixation device of a feeding fence.

In an embodiment, the data acquisition system is a three-dimensional camera system configured to record three-dimensional images of at least the part of the barrel of the cow, such as e.g. the back. The camera system is arranged to record images of at least a part of the barrel when the cow is positioned in the detection area.

The recording of images may comprise recording three-dimensional images of at least a part of a top side of the barrel of the cow. In such embodiment, the three-dimensional camera system may be arranged above the detection area to record three-dimensional images of at least a part of a top side of the cow. This position of the camera system above the detection area may be advantageous since the camera system will less interfere with the provision of other equipment, for example a milking robot or feeding device. Also, at this height the chance that dirt will prevent proper functioning of the camera system is substantially reduced.

The three dimensional camera system may be configured to record images at a certain imaging frequency. These images may be used to determine different cow related aspects. This imaging frequency may however be substantially higher than the frequency of images needed to reliably determine the at least one lameness parameter. When all images recorded at the imaging frequency of the camera system are processed to determine the at least one lameness parameter, this will require a substantially higher processing effort. Therefore, the images may be processed with a lower frequency, i.e. less images are used, in order to use less processing power. The frequency of images used for processing may for example be less than 10 Hz, for example 8 Hz.

The frequency of the images used to determine the at least one lameness parameter may be adapted in dependence of the value of the at least one lameness parameter. For example, when the value approaches a threshold value, the frequency may be increased to more precisely determine the at least one lameness parameter. As an alternative, or in addition, the processing of the images may be improved, for example intensified, to more precisely determine the at least one lameness parameter.

According to the invention, the at least one lameness parameter comprises a movement, speed and/or acceleration of the one or more reference locations of the barrel.

When a cow suffers from lameness it may typically make more and faster movements. These faster movements in particular occur because the animal will correct it's standing posture, because it will try to unburden the painful hoof. By doing so the barrel of the cow will make relative fast movements between different positions. Such postural sway may be detected by the method of the invention.

The increase in movements of the barrel of the cow suffering from lameness and the speed of these movements can be determined by determining the acceleration of the barrel when standing in the detection area. Also, the speed of the barrel of such cow making more and faster movements due to lameness may be used as a parameter indicating that the cow suffers from lameness.

A further example of a lameness parameters that may be used to detect that a cow may suffer from lameness may be a ratio, within the time period, between the time the cow is standing still and the time of movement of the cow. When a cow suffers from lameness, it will typically be restless. Thus, when a ratio between standing still and movement is below a threshold value, there is a substantial chance that the cow suffers from lameness.

Another parameter based on movements of the cow is the ratio between time the cow is standing still and the time of continuous movement of the cow. A high ratio indicates that the cow may suffer from lameness.

To determine whether the cow may suffer from lameness, a determined value of the lameness parameter may be compared with a lameness threshold value.

During acquisition of position related data of the barrel, the cow should be held in a detection area in which it is free to make some movements, but within the field of view of the data acquisition system such that position related data of the relevant barrel part can be obtained.

According to the invention, the one or more reference locations include a center of mass of the barrel of the cow, a back end of the barrel, a position of pin bones, hook bones or tail head of the cow, and/or a location of spine.

Any other reference location of the barrel which may be detected on the basis of the position related data, or reference location that can be determined, in particular calculated on the basis of this position related data may also be used.

In an embodiment, the method comprises the step of providing a warning signal when a value of the at least one lameness parameter exceeds a lameness threshold value. The at least one lameness parameter may be indicative whether or not a cow is suffering from lameness. To properly treat a cow, a warning signal is provided when the value of the at least one lameness parameter exceeds a lameness threshold value. This warning signal may for instance be an indicator in a herd management system, or a message that is sent to a central processing device and/or a handheld device, such as a mobile phone or tablet device.

When a warning signal is given, the respective cow may be examined for lameness and, when needed, be treated.

The lameness threshold value may be an absolute threshold value, but also an individual threshold value dependent on the individual characteristics of the cows.

In an embodiment, the method comprises the step of providing an identity of a cow in the detection area, determined by a cow identification system. The provided identity can be associated with the position related data. This allows for instance to determine individual threshold values and/or to store the position related data of a specific cow in a storage device in association with the identity to the respective cow.

In an embodiment, the method comprises the step of comparing the at least one lameness parameter with one or more lameness parameters of the barrel determined with respect to other time periods in which the same cow was present in the detection area in order to monitor development of lameness of the cow in the course of time. The changes of the at least lameness parameter from one individual cow over time could be an indication of development of lameness of that cow. By regularly determining the value of the at least one lameness parameter, for example daily or weekly, the development of lameness, for example recovery of lameness, can be monitored.

The monitoring may be based on automatic lameness detection according to the method of the invention. However, also other lameness detection steps may be used in combination with the automatic lameness parameter detection according to the invention. For example, a farmer may see that one of the cows suffers from lameness. In such case, the lameness detection may be entered into the processing device for instance via a herd management system.

In an embodiment, the method comprises the step of providing an identity of a cow in the detection area, determined by a cow identification system. By using a cow identification system, the cow entering the detection area can be identified. When the identity of a cow is established and provided to the processing device, the at least one lameness parameter can be automatically associated with the identity of the cow.

In an embodiment, the method comprises the step of storing the determined at least one lameness parameter in a storage device of the processing device. The determined at least one lameness parameter may be stored in a storage device of the processing device in combination with the identity of the cow.

In an embodiment, the method comprises the step of comparing the at least one lameness parameter with one or more lameness parameters of the barrel determined during previous time periods in which the same cow was present in the detection area in order to monitor development of lameness of the cow in the course of time. The information stored in the storage device may for example be used to monitor the development of lameness of an individual cow, but may also be used as statistical data. In the latter case, or in other circumstances, the at least one lameness parameter may also be stored without being associated to a specific cow. For example, it may be interesting to monitor the occurrence of lameness in a herd of cows.

In an embodiment, the detection area is a milking box of a milking robot, wherein the time period is at least a part of a milking time of the cow.

The invention further provides a lameness detection device configured to detect lameness of a cow, according to claim 8.

In an embodiment, the lameness detection device comprises a cow identification system configured to determine an identity of a cow entering or being present in the confinement area.

In an embodiment, the processing device comprises a storage device to store the at least one lameness parameter.

In an embodiment, the processing device is configured to provide a warning signal when a value of the at least one lameness parameter exceeds a lameness threshold value.

In an embodiment, the processing device is configured to compare the at least one lameness parameter with one or more lameness parameters of the cow determined during previous time periods in which the cow was present in the confinement area in order to monitor development of lameness of the cow in the course of time.

Embodiments of a method and arrangement according to the invention will now be described in further detail, by way of example only, with reference to the accompanying drawings, in which:

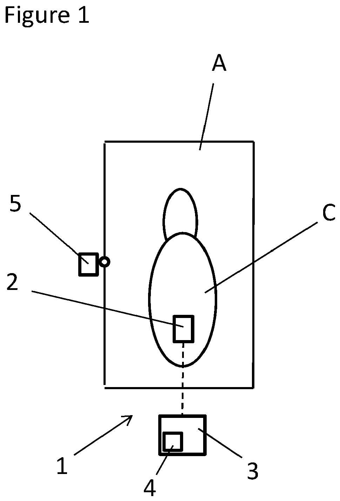

Figure 1 shows a top view of a device to detect lameness of a cow.

Figure 1 shows a top view of a detection area A, in which a cow C is present. The detection area A is for example a milking box of an automatic milking machine or a milking robot. The cow C will typically stay within such milking box for a time period of 5-15 minutes. In the detection area A, the cow C is confined in such a way that movements of the cow are limited. The cow may make some small movements, such a take a step forward or back, a postural sway, etc. However, the cow cannot move over a larger distance, for example cannot walk.

A lameness detection device 1 is provided to detect a lameness parameter indicative of the lameness of a cow. The lameness detection device 1 comprises a three-dimensional camera system 2 configured to record three-dimensional images of at least a part of a body of the cow C present in the detection area A. The camera system 2 is arranged above the detection area A, i.e. at a higher level than the top side of the cow, to record three-dimensional images of at least the top side of the barrel of the cow.

The three-dimensional camera system 2 may comprise any suitable three dimensional camera such as stereoscopic or time of flight 3D camera system.

The camera system 2 may be provided for example at least 2 m above floor level. The camera system 2 is configured to record three-dimensional images of the cow C, whereby the cow C may be in any position within the detection area A.

In the shown embodiment the camera system 2 is arranged at a fixed position, whereby the field of view of the camera system 2 covers practically the whole detection area A. In other embodiments, multiple camera systems 2 may be provided that together cover the area A.

The lameness detection device 1 further comprises a processing device 3 configured to process the images of the cow that are recorded by the camera system 2. The processing device 3 is configured to determine a position of a number of reference location on the barrel of the cow for each of the images of the cow, and to determine on the basis of the determined reference locations at least one lameness parameter indicative of the lameness of the cow.

The processing device 3 may be any suitable device for processing the three dimensional images taken by the three-dimensional camera system 2, and may be a separate device or integrated in the three-dimensional camera system 2. The processing device 3 may also be integrated in a central processing device, for example a central computer device configured to monitor a number of cows, such as a herd management system, and/or configured to monitor and control one or more automatic feeding devices.

The processing device 3 comprises a storage device 4 to store the at least one lameness parameter determined by the processing device 3. The storage device 3 is for example a disk drive of a computer. The storage device 3 may also be used to store other relevant information.

The lameness detection device 1 comprises a cow identification system 5 configured to determine an identity of a cow entering or being present in the detection area A. For this reason the cow C may carry a transponder that allows the cow identification system 5 to determine the identity of the cow C being present in or entering into the detection area A.

When a cow enters the detection area A, the camera system 2 will automatically start recording three-dimensional images of at least a part of a top side of the body of the cow C present in the detection area A.

The processing device 3 processes these three-dimensional images. This processing comprises determining one or more reference locations of the barrel of the cow for a number of images of the cow C, that is used to determine the at least one lameness parameter. On the basis of the reference locations of the barrel of the cow at least one lameness parameter indicative of the lameness of the cow may be calculated.

The at least one lameness parameter comprises for example a movement of the barrel of the cow. It has been found that a cow will typically make more and faster movements with its barrel when it suffers from lameness. These movements in particular occur because the animal will continuously have the need to correct it's standing posture, because it will try to unburden the painful hoof.

Also, the cow C may lean against a sidewall of the detection area A to relieve the foot that causes pain. Such position in which a cow leans against the side wall may also be detected by the lameness detection device 1 during the processing of the three-dimensional images.

The recording of images is carried out during a certain time period. When the detection area A is a milking box of a milking machine in particular a milking robot, the cow will normally stay within the detection area A for a period of for example 5-15 minutes. This time period will normally be sufficient to determine whether it is likely that the cow suffers from lameness.

The imaging frequency with which the three-dimensional images are recorded may for example be 30 frames per second. It is has however been found that processing of images with a lower image processing frequency, i.e. the frequency of the images used for determining the lameness parameter, than the imaging frequency of the camera system 2, may also be sufficient to reliably determine whether the cow suffers from lameness.

For example, images provided at a frequency of 8 Hz may be sufficient to determine a reliable lameness parameter. Thus processing images at this lower frequency may be preferred as it will require less processing power of the processing device 3.

In case the three-dimensional camera system 2 is only used to determine the lameness parameter, the imaging frequency may also be lowered to the image processing frequency, of for example 8 Hz. However, the three dimensional images may also be used for determining other cow-related parameters. When these other cow-related parameters require a higher imaging frequency, the higher imaging frequency of the camera system 2 may be used, and only a limited part of the images may be processed to determine the at least one lameness parameter.

When a value of the at least one lameness parameter determined by the processing device 3 exceeds a certain threshold value, it may be determined that the cow may suffer from lameness. To take appropriate action, a warning signal is provided to warn the farmer. The farmer and/ or a veterinarian may for example check the cow and decide whether the cow should be treated for lameness.

The warning signal may for example be a signal provided by the processing device 3 and displayed in a herd management system to indicate that the respective cow should be checked for lameness. The warning signal could also be in the form of a message to a mobile device of the farmer, such as a mobile telephone or tablet device to indicate that the cow may suffer from lameness.

When a cow identification system 5 is used to determine the identity of the cow C, the lameness parameter that is determined with respect to this cow C may be automatically stored in the storage device 4 in a storage location associated with this cow C.

The storage of this information allows the automatic monitoring of the development of the lameness of a cow. The monitoring for instance comprises comparing the at least one lameness parameter that is determined by the processing device 3 with one or more previous lameness parameters of the cow determined during previous time periods in which the cow was present in the detection area. These one or more previous lameness parameters may be collected from the storage device 4.

The monitoring of the development of lameness may also comprise extra attention for a cow which has been diagnosed with lameness or which is suspected to suffer from lameness. For instance, when the lameness parameter determined by the lameness detection device slowly grows towards the threshold value to provide a warning signal, the processing device may be configured to more intensively process the images to search for parameters indicative for lameness.

Also when a cow is suffering from lameness, the cow C may be monitored more carefully by the lameness detection device 1 in order to determine whether the cow C recovers.

## Claims

1. A method to automatically detect lameness of a cow, comprising the steps of:
- providing position related data, acquired by a data acquisition system, of at least a part of a barrel of a cow kept for a time period within a detection area,
- processing, by a processing device, the position related data of the barrel, wherein processing of the data comprises:
- determining a position, or a derivative thereof, of one or more reference locations of the barrel on the basis of the position related data, wherein the one or more reference locations include a center of mass of the barrel of the cow, a back end of the barrel, a position of pin bones, hook bones or tail bones of the cow, and/or a location of the spine,
- determining on the basis of the determined position, or the derivative thereof, of the one or more reference locations at least one lameness parameter indicative of the lameness of the cow,
**characterised in that** the at least one lameness parameter comprises a movement, speed and/or acceleration of the one or more reference locations of the barrel.

2. The method of claim 1, wherein the data acquisition system is a three-dimensional camera system configured to record three-dimensional images of at least the part of the barrel of the cow.

3. The method of any of the preceding claims, wherein the method comprises the step of providing a warning signal when a value of the at least one lameness parameter exceeds a lameness threshold value.

4. The method of any of the preceding claims, wherein the method comprises the step of providing an identity of a cow in the detection area, determined by a cow identification system.

5. The method of any of the preceding claims, wherein the method comprises the step of storing the determined at least one lameness parameter in a storage device of the processing device.

6. The method of any of the preceding claims, wherein the method comprises the step of comparing the at least one lameness parameter with one or more lameness parameters of the barrel determined with respect to other time periods in which the same cow was present in the detection area in order to monitor development of lameness of the cow in the course of time.

7. The method of any of the preceding claims, wherein the detection area is a milking box of a milking robot, and wherein the time period is at least a part of a milking time of the cow.

8. A lameness detection device configured to detect lameness of a cow, comprising:
- a data acquisition system configured to record position related data of at least a part of a barrel of the cow present in a detection area,
- a processing device configured to process the position related data, said processing comprises the steps of:
- determining a position, or a derivative thereof, of one or more reference locations of the barrel on the basis of the position related data, wherein the one or more reference locations include a center of mass of the barrel of the cow, a back end of the barrel, a position of pin bones, hook bones or tail bones of the cow, and/or a location of the spine, and
- determining on the basis of the determined position, or the derivative thereof, of the one or more reference locations at least one lameness parameter indicative of the lameness of the cow,
**characterised in that** the at least one lameness parameter comprises a movement, speed and/or acceleration of the one or more reference locations of the barrel

9. The lameness detection device of claim 8, wherein the data acquisition system is a three-dimensional camera system configured to record three-dimensional images of at least the part of the barrel.

10. The lameness detection device of claim 8 or 9, wherein the lameness detection device comprises a cow identification system configured to determine an identity of a cow entering or being present in the detection area.

11. The lameness detection device of any of the claims 8-10, wherein the processing device comprises a storage device to store the at least one lameness parameter.

12. The lameness detection device of any of the claims 8-11, wherein the processing device is configured to provide a warning signal when a value of the at least one lameness parameter exceeds a lameness threshold value.

13. The lameness detection device of any of the claims 8-12, wherein the processing device is configured to compare the at least one lameness parameter with one or more lameness parameters of the cow determined with respect to other time periods in which the cow was present in the detection area in order to monitor development of lameness of the cow in the course of time.

## Patentansprüche

1. Verfahren zum automatischen Detektieren von Lahmheit einer Kuh, das die folgenden Schritte umfasst:
- Bereitstellen von positionsbezogenen Daten, erfasst durch ein Datenerfassungssystem, von zumindest einem Teil eines Rumpfes einer Kuh, die eine Zeitspanne lang innerhalb des Detektionsbereichs gehalten wird,
- Verarbeiten, durch eine Verarbeitungsvorrichtung, der positionsbezogenen Daten des Rumpfes, wobei Verarbeitung der Daten Folgendes umfasst:
- Bestimmen einer Position, oder einer Ableitung davon, von einer oder mehreren Bezugspositionen des Rumpfes auf Grundlage der positionsbezogenen Daten, wobei die eine oder die mehreren Bezugspositionen einen Massemittelpunkt des Rumpfes der Kuh, ein hinteres Ende des Rumpfes, eine Position der Sitzbeinhöcker, Hüfthöcker oder Schwanzknochen der Kuh und/oder eine Position der Wirbelsäule umfassen,
- Bestimmen, auf Grundlage der bestimmten Position, oder der Ableitung davon, der einen oder der mehreren Bezugspositionen zumindest eines Lahmheitsparameters, der indikativ für die Lahmheit der Kuh ist,
**dadurch gekennzeichnet, dass** der zumindest eine Lahmheitsparameter eine Bewegung, Geschwindigkeit und/oder Beschleunigung der einen oder mehreren Bezugspositionen des Rumpfes umfasst.

2. Verfahren nach Anspruch 1, wobei das Datenerfassungssystem ein dreidimensionales Kamerasystem ist, das ausgelegt ist zum Aufzeichnen von dreidimensionalen Bildern zumindest des Teils des Rumpfes der Kuh.

3. Verfahren nach einem der vorhergehenden Ansprüche, wobei das Verfahren den Schritt des Bereitstellens eines Warnsignals umfasst, wenn ein Wert des zumindest einen Lahmheitsparameters einen Lahmheitsschwellenwert überschreitet.

4. Verfahren nach einem der vorhergehenden Ansprüche, wobei das Verfahren den Schritt des Bereitstellens einer Identität einer Kuh im Detektionsbereich bereitstellt, bestimmt durch ein Kuhidentifizierungssystem.

5. Verfahren nach einem der vorhergehenden Ansprüche, wobei das Verfahren den Schritt des Speicherns des bestimmten zumindest einen Lahmheitsparameters in einer Speichervorrichtung der Verarbeitungsvorrichtung umfasst.

6. Verfahren nach einem der vorhergehenden Ansprüche, wobei das Verfahren den Schritt des Vergleichens des zumindest einen Lahmheitsparameters mit einem oder mehreren Lahmheitsparametern des Rumpfes umfasst, die bezüglich anderer Zeitspannen bestimmt wurden, in denen die gleiche Kuh im Detektionsbereich anwesend war, um die Entwicklung von Lahmheit der Kuh im Laufe der Zeit zu überwachen.

7. Verfahren nach einem der vorhergehenden Ansprüche, wobei der Detektionsbereich eine Melkbox eines Melkroboters ist und wobei die Zeitspanne zumindest ein Teil einer Melkzeit der Kuh ist.

8. Lahmheitsdetektionsvorrichtung, ausgelegt zum Detektieren von Lahmheit einer Kuh, die Folgendes umfasst:
- ein Datenerfassungssystem, ausgelegt zum Aufzeichnen von positionsbezogenen Daten zumindest eines Teils eines Rumpfes der im Detektionsbereich anwesenden Kuh,
- eine Verarbeitungsvorrichtung, ausgelegt zum Verarbeiten der positionsbezogenen Daten, wobei die Verarbeitung die folgenden Schritte umfasst:
- Bestimmen einer Position, oder einer Ableitung davon, von einer oder mehreren Bezugspositionen des Rumpfes auf Grundlage der positionsbezogenen Daten, wobei die eine oder die mehreren Bezugspositionen einen Massemittelpunkt des Rumpfes der Kuh, ein hinteres Ende des Rumpfes, eine Position der Sitzbeinhöcker, Hüfthöcker oder Schwanzknochen der Kuh und/oder eine Position der Wirbelsäule umfassen, und
- Bestimmen, auf Grundlage der bestimmten Position, oder der Ableitung davon, der einen oder mehreren Bezugspositionen zumindest eines Lahmheitsparameters, der indikativ für die Lahmheit der Kuh ist,
**dadurch gekennzeichnet, dass** der zumindest eine Lahmheitsparameter eine Bewegung, Geschwindigkeit und/oder Beschleunigung der einen oder der mehreren Bezugspositionen des Rumpfes umfasst.

9. Lahmheitsdetektionsvorrichtung nach Anspruch 8, wobei das Datenerfassungssystem ein dreidimensionales Kamerasystem ist, das ausgelegt ist zum Aufzeichnen von dreidimensionalen Bildern zumindest des Teils des Rumpfes.

10. Lahmheitsdetektionsvorrichtung nach Anspruch 8 oder 9, wobei die Lahmheitsdetektionsvorrichtung ein Kuhidentifizierungssystem umfasst, das ausgelegt ist zum Bestimmen einer Identität einer Kuh, die in den Detektionsbereich eintritt oder in diesem anwesend ist.

11. Lahmheitsdetektionsvorrichtung nach einem der Ansprüche 8-10, wobei die Verarbeitungsvorrichtung eine Speichervorrichtung zum Speichern des zumindest einen Lahmheitsparameters umfasst.

12. Lahmheitsdetektionsvorrichtung nach einem der Ansprüche 8-11, wobei die Verarbeitungsvorrichtung ausgelegt ist zum Bereitstellen eines Warnsignals, wenn ein Wert des zumindest einen Lahmheitsparameters einen Lahmheitsschwellenwert überschreitet.

13. Lahmheitsdetektionsvorrichtung nach einem der Ansprüche 8-12, wobei die Verarbeitungsvorrichtung ausgelegt ist zum Vergleichen des zumindest einen Lahmheitsparameters mit einem oder mehreren Lahmheitsparametern der Kuh, die bezüglich anderer Zeitspannen bestimmt wurden, in denen die Kuh im Detektionsbereich anwesend war, um die Entwicklung von Lahmheit der Kuh im Laufe der Zeit zu überwachen.

## Revendications

1. Procédé de détection automatique d'une boiterie chez une vache, comprenant les étapes suivantes :
- la fourniture de données de position, acquises par un système d'acquisition de données, relatives à au moins une partie d'un tronc d'une vache maintenue pendant une période de temps au sein d'une zone de détection,
- le traitement, par un dispositif de traitement, des données de position relatives au tronc, le traitement des données comprenant :
- la détermination d'une position, ou d'une dérivée de celle-ci, d'un ou de plusieurs emplacements de référence du tronc sur la base des données de position, les un ou plusieurs emplacements de référence comportant un centre de gravité du tronc de la vache, une extrémité arrière du tronc, une position de pointes de fesses, de pointes de hanches ou de coccyx de la vache, et/ou un emplacement de la colonne vertébrale,
- la détermination, sur la base de la position déterminée, ou de la dérivée de celle-ci, des un ou plusieurs emplacements de référence, d'au moins un paramètre de boiterie indiquant la boiterie chez la vache,
**caractérisé en ce que** l'au moins un paramètre de boiterie comprend un déplacement, une vitesse et/ou une accélération des un ou plusieurs emplacements de référence du tronc.

2. Procédé selon la revendication 1, dans lequel le système d'acquisition de données est un système de caméra tridimensionnelle configuré pour enregistrer des images tridimensionnelles d'au moins la partie du tronc de la vache.

3. Procédé selon l'une quelconque des revendications précédentes, le procédé comprenant l'étape de fourniture d'un signal avertisseur lorsqu'une valeur de l'au moins un paramètre de boiterie dépasse une valeur-seuil de boiterie.

4. Procédé selon l'une quelconque des revendications précédentes, le procédé comprenant l'étape de fourniture d'une identité d'une vache dans la zone de détection, déterminée par un système d'identification de vache.

5. Procédé selon l'une quelconque des revendications précédentes, le procédé comprenant l'étape de stockage de l'au moins un paramètre de boiterie déterminé dans un dispositif de stockage du dispositif de traitement.

6. Procédé selon l'une quelconque des revendications précédentes, le procédé comprenant l'étape de comparaison de l'au moins un paramètre de boiterie à un ou plusieurs paramètres de boiterie du tronc déterminés par rapport à d'autres périodes de temps au cours desquelles la même vache était présente dans la zone de détection dans le but de surveiller l'évolution d'une boiterie chez la vache au fil du temps.

7. Procédé selon l'une quelconque des revendications précédentes, dans lequel la zone de détection est une stalle de traite d'un robot de traite, et dans lequel la période de temps est au moins une partie d'un temps de traite de la vache.

8. Dispositif de détection de boiterie configuré pour détecter une boiterie chez une vache, comprenant :
- un système d'acquisition de données configuré pour enregistrer des données de position relatives à au moins une partie d'un tronc d'une vache présente dans une zone de détection,
- un dispositif de traitement configuré pour traiter les données de position, ledit traitement comprenant les étapes suivantes :
- la détermination d'une position, ou d'une dérivée de celle-ci, d'un ou de plusieurs emplacements de référence du tronc sur la base des données de position, les un ou plusieurs emplacements de référence comportant un centre de gravité du tronc de la vache, une extrémité arrière du tronc, une position de pointes de fesses, de pointes de hanches ou de coccyx de la vache, et/ou un emplacement de la colonne vertébrale, et
- la détermination, sur la base de la position déterminée, ou de la dérivée de celle-ci, des un ou plusieurs emplacements de référence, d'au moins un paramètre de boiterie indiquant la boiterie chez la vache,
**caractérisé en ce que** l'au moins un paramètre de boiterie comprend un déplacement, une vitesse et/ou une accélération des un ou plusieurs emplacements de référence du tronc.

9. Dispositif de détection de boiterie selon la revendication 8, dans lequel le système d'acquisition de données est un système de caméra tridimensionnelle configuré pour enregistrer des images tridimensionnelles d'au moins la partie du tronc.

10. Dispositif de détection de boiterie selon la revendication 8 ou 9, le dispositif de détection de boiterie comprenant un système d'identification de vache configuré pour déterminer une identité d'une vache qui pénètre dans la zone de détection ou qui y est présente.

11. Dispositif de détection de boiterie selon l'une quelconque des revendications 8 à 10, dans lequel le dispositif de traitement comprend un dispositif de stockage pour stocker l'au moins un paramètre de boiterie.

12. Dispositif de détection de boiterie selon l'une quelconque des revendications 8 à 11, dans lequel le dispositif de traitement est configuré pour fournir un signal avertisseur lorsqu'une valeur de l'au moins un paramètre de boiterie dépasse une valeur-seuil de boiterie.

13. Dispositif de détection de boiterie selon l'une quelconque des revendications 8 à 12, dans lequel le dispositif de traitement est configuré pour comparer l'au moins un paramètre de boiterie à un ou plusieurs paramètres de boiterie de la vache déterminés par rapport à d'autres périodes de temps au cours desquelles la vache était présente dans la zone de détection dans le but de surveiller l'évolution d'une boiterie chez la vache au fil du temps.
